# EUROPÄISCHE PATENTSCHRIFT

(11) **EP 0 761 655 B1**
(45) Veröffentlichungstag und Bekanntmachung des Hinweises auf die Patenterteilung: **02.06.1999**
(21) Anmeldenummer: 96113727.0
(22) Anmeldetag: 28.08.1996
(51) Int. Cl.: C07D 263/22, C07C 269/06

(54) **Verfahren zur Herstellung von N-Alkenylcarbaminsäureestern**
Process for the preparation of N-alkenylcarbamic acid esters
Procédé de préparation d'ester d'acide N-alkenylcarbamique

(30) Priorität: 08.09.1995 DE 19533219
(43) Veröffentlichungstag der Anmeldung: 12.03.1997
(73) Patentinhaber: BASF AKTIENGESELLSCHAFT, 67056 Ludwigshafen (DE)
(72) Erfinder: Henkelmann, Jochem, Dr., 68165 Mannheim (DE); Heider, Marc, Dr., 67433 Neustadt (DE); Rühl, Thomas, Dr., 67227 Frankenthal (DE)

(56) Entgegenhaltungen:
- EP-A- 0 351 603
- EP-A- 0 703 219
- GB-A- 851 773
- US-A- 5 233 077
- CHEMICAL ABSTRACTS, vol. 100, no. 3, 16.Januar 1984 Columbus, Ohio, US; abstract no. 22611k, XP002019667 & KHIM. PRIR. SOEDIN., Bd. 4, 1983, Seiten 507-511, R.G. AFLYATUNOVA ET AL.:
- "BEILSTEINS HANDBUCH DER ORGANISCHEN CHEMIE" 1978 , SPRINGER-VERLAG , BERLIN XP002019666 * Band 6, 4. Ergänzungswerk, Seite 631 *
- "BEILSTEINS HANDBUCH DER ORGANISCHEN CHEMIE" 1979 , SPRINGLER-VERLAG , BERLIN XP002019733 * Band 4, 4. Ergänzungswerk, Seiten 1054-1055 *

## Beschreibung

Die vorliegende Erfindung betrifft ein neues Verfahren zur Herstellung von N-Alkenylcarbaminsäureestern der allgemeinen Formel I 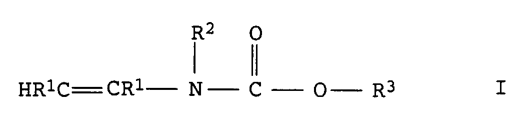 in der die Reste R¹ Wasserstoff bedeuten, die Reste R² und R³ für eine C₁-C₂₀-Alkylgruppe stehen oder gemeinsam ein 2- bis 7-gliedriges Alkylenbrückenglied bilden, aus einem Carbonsäurealkenylester der allgemeinen Formel II 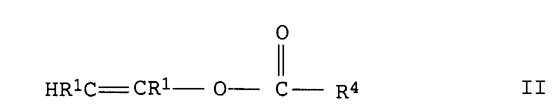 in der R¹ die oben angegebene Bedeutung hat und R⁴ für Wasserstoff, C₁- bis C₂₀-Alkylgruppe, oder einen cycloaliphatischen Rest mit 4 bis 7 Kohlenstoffatomen steht, und einem Carbaminsäureester der allgemeinen Formel III 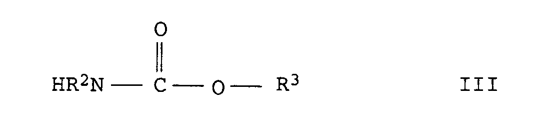 in der die Reste R² und R³ die oben angegebene Bedeutung haben, dadurch gekennzeichnet, daß man die Ausgangsverbindungen in Gegenwart eines tertiären Amins umsetzt.

Die Verfahrensprodukte der Formel I sind gesuchte Zwischenprodukte zur Synthese von Spezialpolymeren (US-A 4 680 410).

In der US-A 4 831 153 wird die Synthese von N-Alkenylcarbaminsäureestern durch Umsetzung der entsprechenden Carbaminsäureester mit Acetaldehyd und Thermolyse der so erhaltenen α-Hydroxyalkylverbindungen zu den N-Alkenylderivaten beschrieben. Eine Variante dieser Methode stellt die Pyrolyse der entsprechenden N-Silyloxyalkyl-Vorstufen dar (US-A 4 680 410).

Diese Verfahren zur Herstellung der N-Alkenylcarbaminsäureester sind zweistufig und erfordern einen thermischen Eliminierungsschritt. Sie sind somit technisch relativ aufwendig und führen aufgrund von Verlusten an Wertprodukt bei den erforderlichen hohen Reaktionstemperaturen nur zu unbefriedigenden Gesamtausbeuten.

Die US-A 5 155 253 und EP-A 506 070 betreffen Verfahren zur Herstellung von N-Alkenylverbindungen aus Alkenylestern und N-substituierten Carbaminsäureestern in Gegenwart von Metallen der Platingruppe, insbesondere Ruthenium, als Katalysator. In technischen Verfahren kommt es dabei zur Reduktion des Katalysators, so daß es erforderlich wird, die teueren Edelmetallverbindungen nachzudosieren.

Es bestand daher die Aufgabe, ein Verfahren bereitzustellen, das die genannten Nachteile bekannter Verfahren vermeidet.

Demgemäß wurde das oben definierte Verfahren zur Herstellung von N-Alkenylcarbaminsäureestern der Formel I gefunden, das dadurch gekennzeichnet ist, daß man die Ausgangsverbindungen in Gegenwart einer Base umsetzt.

Die nachstehende Reaktionsgleichung verdeutlicht das beanspruchte Verfahren am Beispiel der Herstellung von N-Vinyloxazolidon aus Vinylacetat und Oxazolidon in Gegenwart von 4-Dimethylpyridin.

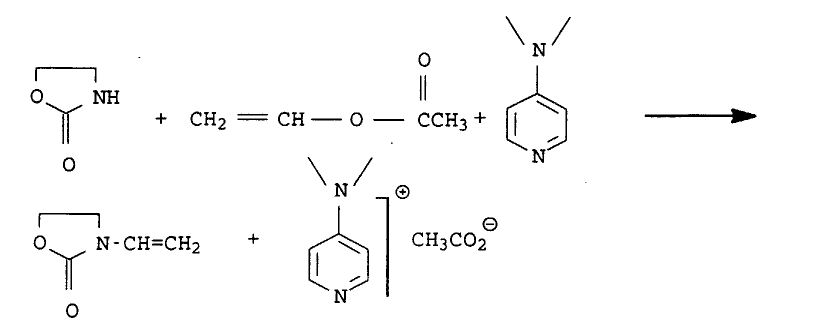

Im erfindungsgemäßen Verfahren wird formal eine Vinylgruppe aus einem Carbonsäurevinylester der Formel II auf einen Carbaminsäureester der Formel III übertragen.

In der Vinylgruppe der Ester der Formel II stehen die Reste R¹ für Wasserstoff. Der Rest R⁴ in den Estern der Formel II steht für C₁-C₂₀-Alkylgruppen wie Methyl, Ethyl, n-Propyl, n-Butyl, tert.-Butyl, Neodecyl und Stearyl. Weiterhin kann R⁴ für cycloaliphatische Reste mit 4 - 7 Kohlenstoffatomen stehen, z.B. Cyclopentyl und Cyclohexyl. Besonders bevorzugt steht R⁴ außerdem für Wasserstoff.

Als Ausgangsverbindungen der Formel II seien genannt: Vinylformiat, Vinylacetat, Vinylpropionat, Vinylstearat, Vinylpivalat und 4-tert.-Butylbenzoylvinylester.

Die Verbindungen der Formel II sind im Handel erhältlich oder nach bekannten Methoden herstellbar, beispielsweise durch Addition von Carbonsäuren an Acetylen oder durch Acetoxilierung von Ethylen (Weissermel u. Arpe, Industrielle Organische Chemie, 2. Auflage, 1978, Verlag Chemie, S. 217ff).

Der Rest R² in den Carbaminsäureestern der Formel III steht für Alkylgruppen, die bevorzugt 1 bis 10 Kohlenstoffatome tragen und geradkettig oder verzweigt sind. Besonders bevorzugt sind C₁-C₄-Alkylgruppen wie Methyl, Ethyl, n-Propyl und n-Butyl.

Der Rest R² kann mit dem Rest R³ weiterhin ein 2- bis 7-gliedriges Brückenglied formen.

Für den Rest R³ der Carbaminsäureester der Formel III gilt das oben zum Rest R⁴ gesagte analog mit dem Unterschied, daß R³ nicht für Wasserstoff steht. Als Ausgangsverbindungen seien genannt: N-Methylcarbaminsäuremethylester, N-Butylcarbaminsäuremethylester, N-Methylcarbaminsäureethylester, N-Methylcarbaminsäurephenylester, N-Propylcarbaminsäurebenzylester und Oxazolidon.

Auch diese Verbindungen sind im Handel oder nach bekannten Methoden erhältlich, z.B. durch Umsetzung von Isocyanaten mit Alkoholen und von primären Aminen mit Alkylchlorformiaten unter HCl-Abspaltung.

Bevorzugte Verfahrensprodukte sind N-Butyl-N-vinylcarbaminsäuremethylester und N-Vinyloxazolidon.

Die Ausgangsverbindungen werden in Gegenwart eines tertiären Amins umgesetzt.

Geeignete tertiäre Amine sind beispielsweise:
Trialkylamine wie Trioctylamin, Ethyldiisopropylamin, Diethylisopropylamin, Dimethylcyclohexylamin, Triethylamin, außerdem cyclische Amine wie 2,2,6,6-Tetramethylpiperidin, 1,4-Diazabicyclo[2.2.2]octan, 1,8-Diazabicyclo[5.4.0]-undec-7-en, aliphatische und aromatische Reste tragende Amine wie 1,8-Bis(dimethylamino)-naphthalin und 4-Dimethylaminopyridin und heterocyclische Amine wie N-Alkylimidazole und N-Arylimidazole.

Pro Äquivalent Carbaminsäureester der Formel III können 0,1 bis 10, vorzugsweise 1 bis 1,2 Äquivalente des Esters der Formel II eingesetzt werden.

Die Basenmenge kann 0,1 bis 3 Äquivalente, bevorzugt 0,2 bis 1 Äquivalent pro Äquivalent Carbaminsäureester der Formel III betragen.

Obwohl die Umsetzung bevorzugt ohne Lösungsmittel ausgeführt wird, kann jedoch ein Lösungsmittel zugesetzt werden, z.B. aprotische Lösungsmittel wie Ether, z.B. Tetrahydrofuran, aromatische Kohlenwasserstoffe wie Toluol und Xylol, Ketone wie Aceton, weiterhin Acetonitril, Hexamethylphosphorsäuretriamid, Sulfolan, Dimethylsulfoxid, Harnstoffe wie N,N'-Dimethylethylen-, N,N'-Dimethylpropylenharnstoff und Tetrabutylharnstoff. Die Menge liegt im allgemeinen bei 10 bis 30 Gew.-%, bezogen auf den Gesamtansatz.

Die Reaktionstemperatur liegt in der Regel bei 0 bis 150°C, bevorzugt bei 20 bis 120°C. Bevorzugt wird bei Normaldruck gearbeitet, es ist aber auch möglich, bei 0,01 bis 10 bar Reaktionsdruck zu arbeiten.

Die Reaktion kann kontinuierlich und diskontinuierlich ausgeführt werden. So können die Ausgangsverbindungen und die Base in einen Rührkessel gegeben und darin umgesetzt werden, wobei die Reihenfolge der Zugabe der Einzelkomponenten keinen erkennbaren Einfluß auf die Reaktion hat. Es ist auch möglich, die Ausgangsverbindungen und die Base in einem Rohrreaktor in Riesel- oder Sumpffahrweise umzusetzen. Es hat sich als vorteilhaft erwiesen, die Reaktion in einem Strahldüsenreaktor vorzunehmen.

In der Regel ist die Reaktion nach 5 Minuten bis 8 Stunden beendet.

Das so erhaltene Reaktionsgemisch kann in an sich bekannter Weise aufgearbeitet werden. Im allgemeinen wird das Produkt destillativ abgetrennt. Der Destillationssumpf kann zur Freisetzung der organischen Basen aus den bei der Reaktion anfallenden Salzen mit Laugen wie Natronlauge versetzt werden. Die freigesetzten Basen können anschließend extraktiv oder destillativ isoliert werden. Werden in der erfindungsgemäßen Umsetzung leichtflüchtige salzartige Verbindungen wie Formiate tertiärer Ammoniumverbindungen gebildet, können diese auch destillativ aufgearbeitet und in die entsprechenden Amine überführt werden. Wird in der erfindungsgemäßen Umsetzung 4-Dimethylaminopyridin eingesetzt, so kann die Base aus dem Destillationssumpf durch Destillation direkt zurückgewonnen werden. Die jeweils abgetrennten Basen können in die Reaktion zurückgeführt werden.

Das erfindungsgemäße Verfahren erlaubt die einstufige Herstellung von N-Alkenylcarbaminsäureestern aus leicht zugänglichen Vorprodukten. Die Reaktion läßt sich technisch einfach gestalten und läuft unter milden Reaktionstemperaturen ab. Sie führt weiterhin zu einer hohen Ausbeute an den Verfahrensprodukten.

### Beispiele

### Herstellung von N-Vinyloxazolidon

### Beispiel 1

43,5 g (0,5 mol) Oxazolidon wurden in 100 ml Tetrahydrofuran suspendiert und mit 50 g (0,5 mol) Triethylamin versetzt. Bei 70 - 73°C wurden dann 52 g (0,6 mol) Vinylacetat innerhalb von 30 Minuten zugetropft. Nach 5 h bei 75°C wurde das Reaktionsgemisch destillativ aufgearbeitet.
Ausbeute: 36,8 g (65%) N-Vinyloxazolidon

### Beispiel 2

In Analogie zu Beispiel 1 wurden 43,5 (0,5 mol) Oxazolidon in 100 g Xylol suspendiert und mit 61,1 g (0,5 mol) 4-Dimethylaminopyridin versetzt. Danach wurden 60 g (0,6 mol) Vinylpropionat bei 105°C innerhalb 1 Stunde zugetropft. Nach 5 Stunden bei 110°C wurde das Reaktionsgemisch destillativ aufgearbeitet.
Ausbeute: 46,9 g (83%) N-Vinyloxazolidon

### Herstellung von N-Butyl-N-Vinylcarbaminsäuremethylester

### Beispiel 3

22,4 g (0,2 mol) N-Butylcarbaminsäuremethylester wurden in 50 g Xylol suspendiert und mit 12 g (0,1 mol) 4-Dimethylaminopyridin versetzt. Danach wurde das Reaktionsgemisch auf 105°C erhitzt und innerhalb von 15 Minuten wurden 22 g (0,2 mol) Vinylpropionat zugetropft. Nach 4 Stunden bei 110°C wurde das Reaktionsgemisch destillativ aufgearbeitet.
Ausbeute: 27,4 g (87%) N-Butyl-N-vinylcarbaminsäuremethylester

## Patentansprüche

1. Verfahren zur Herstellung von N-Alkenylcarbaminsäureestern der allgemeinen Formel I 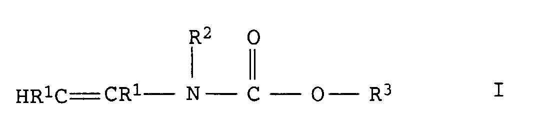 in der die Reste R¹ Wasserstoff bedeuten, die Reste R² und R³ für eine C₁-C₂₀-Alkylgruppe stehen oder gemeinsam ein 2- bis 7-gliedriges Alkylenbrückenglied bilden, aus einem Carbonsäurealkenylester der allgemeinen Formel II 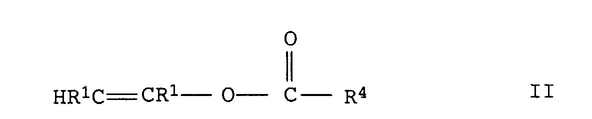 in der R¹ die oben angegebene Bedeutung hat und R⁴ für Wasserstoff, C₁- bis C₂₀-Alkylgruppe, oder einen cycloaliphatischen Rest mit 4 bis 7 Kohlenstoffatomen steht, und einem Carbaminsäureester der allgemeinen Formel III 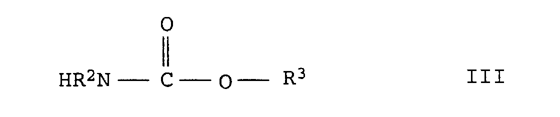 in der die Reste R² und R³ die oben angegebene Bedeutung haben, dadurch gekennzeichnet, daß man die Ausgangsverbindungen in Gegenwart eines tertiären Amins umsetzt.

2. Verfahren nach Anspruch 1, dadurch gekennzeichnet, daß man N-Vinyloxazolidon oder N-Vinyl-N-butylcarbaminsäuremethylester herstellt.

3. Verfahren nach Anspruch 1 oder 2, dadurch gekennzeichnet, daß man die Umsetzung ohne Lösungsmittel vornimmt.

4. Verfahren nach den Ansprüchen 1 bis 3, dadurch gekennzeichnet, daß man 0,2 bis 1 Äquivalent tertiäres Amin pro Äquivalent Carbaminsäureester der Formel III verwendet.

5. Verfahren nach den Ansprüchen 1 bis 4, dadurch gekennzeichnet, daß man die Umsetzung bei 20 bis 80°C vornimmt.

## Claims

1. A process for preparing N-alkenylcarbamic esters of the general formula I 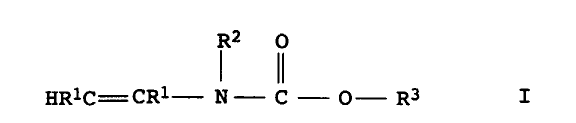 where the R¹ radicals are hydrogen, the R² and R³ radicals are C₁-C₂₀-alkyl or together form a 2- to 7-membered alkylene bridge, from an alkenyl carboxylate of the general formula II 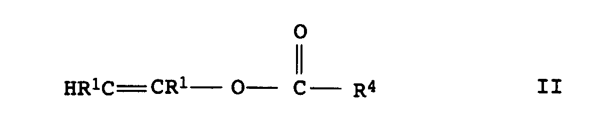 where R¹ has the abovementioned meaning, and R⁴ is hydrogen, C₁-C₂₀-alkyl, or a cycloaliphatic radical having 4 to 7 carbon atoms, and a carbamic ester of the general formula III 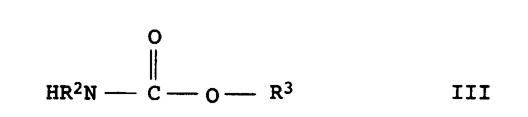 where the R² and R³ radicals have the abovementioned meanings, wherein the starting compounds are reacted in the presence of a tertiary amine.

2. A process as claimed in claim 1, wherein N-vinyloxazolidone or methyl N-vinyl-N-butylcarbamate is prepared.

3. A process as claimed in claim 1 or 2, wherein the reaction is carried out without solvent.

4. A process as claimed in any of claims 1 to 3, wherein 0.2 to 1 equivalent of tertiary amine is used per equivalent of carbamic ester of the formula III.

5. A process as claimed in any of claims 1 to 4, wherein the reaction is carried out at 20 to 80°C.

## Revendications

1. Procédé de préparation d'esters d'acides N-alcénylcarbamique de formule générale I 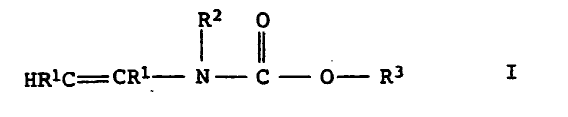 dans laquelle les restes R¹ représentent des atomes d'hydrogène, les restes R² et R³ sont mis pour des groupements alkyle en C₁-C₂₀ ou bien forment ensemble un pont alkylène à 2-7 maillons, à partir d'un ester alcénylique d'acide carboxylique de formule générale II 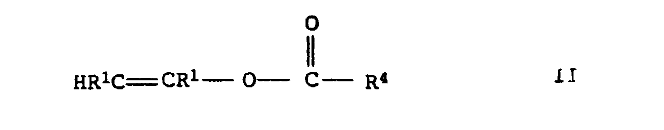 dans laquelle R¹ prend la signification susmentionnée et R⁴ est mis pour un atome d'hydrogène, un groupement alkyle en C₁-C₂₀, ou un reste cycloaliphatique à 4-7 atomes de carbone, et d'un ester d'acide carbamique de formule générale III 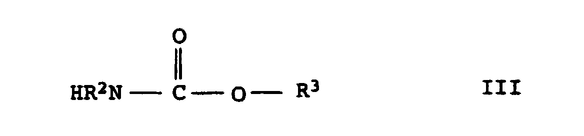 dans laquelle les restes R² et R³ prennent les significations susmentionnées, caractérisé en ce que l'on fait réagir les composés de départ en présence d'une amine tertiaire.

2. Procédé selon la revendication 1, caractérisé en ce que l'on prépare la N-vinyloxazolidone ou l'ester méthylique d'acide N-vinyl-N-butylcarbamique.

3. Procédé selon la revendication 1 ou 2, caractérisé en ce que l'on mène la réaction sans solvant.

4. Procédé selon l'une quelconque des revendications 1 à 3, caractérisé en ce que l'on utilise 0 2-1 équivalent d'amine tertiaire par équivalent d'ester d'acide carbamique de formule III.

5. Procédé selon l'une quelconque des revendications 1 à 4, caractérisé en ce que l'on mène la réaction à 20-80°C.
